# EUROPEAN PATENT APPLICATION

(11) **EP 4 005 382 A1**
(43) Date of publication of application: **01.06.2022**
(21) Application number: 20842935.7
(22) Date of filing: 10.07.2020
(51) Int. Cl.: A01N 1/02, A61K 38/48, A61K 38/49, A61P 7/08, A61P 43/00

(54) **PERFUSION FLUID AND PERFUSION METHOD**

(30) Priority: 23.07.2019 JP 2019135035
(71) Applicant: SCREEN Holdings Co., Ltd., Kyoto 602-8585 (JP)
(72) Inventor: OHARA Masayuki, Kyoto-shi, Kyoto 602-8585 (JP); YOSHIMOTO Syuhei, Kyoto-shi, Kyoto 602-8585 (JP)
(74) Representative: Kilian Kilian & Partner
(86) International application number: PCT/JP2020/026969
(87) International publication number: WO 2021/014997

(57) **Abstract**

A perfusion method includes a) cleaning a blood vessel of an organ by causing a first perfusate to flow into the blood vessel and letting the first perfusate out of the organ through the blood vessel, and b) after the operation a), preserving the organ while causing a second perfusate to flow into the blood vessel and letting the second perfusate out of the organ through the blood vessel. The first perfusate and the second perfusate each contain a biocompatible solution and a serine protease and have an osmotic pressure ratio higher than or equal to 1 and lower than or equal to 5 relative to the saline solution. The concentration of the serine protease in the second perfusate is lower than that in the first perfusate and is higher than or equal to 0.01 mg/mL and lower than or equal to 100 mg/mL. Accordingly, it is possible to dissolve thrombi in the blood vessel of the organ or prevent the formation of new thrombi during organ perfusion treatment.

## Description

### Technical Field

The present invention relates to a perfusate for use in organ perfusion treatment and an organ perfusion method using the perfusate.

### Background Art

In transplant operations such as liver transplantation, an organ from a donor is preserved temporarily until the organ is transplanted into a recipient. Various methods have been developed in order to preserve an organ in a transplantable condition. For example, a perfusion preservation method is known in which intraorgan vascular networks are perfused with a perfusate (e.g., a UW solution) for the purpose of removing waste products in an organ.

However, in cases such as where an organ is preserved ex vivo for a long time or where a considerable amount of time has already elapsed since the bloodstream in vivo was stopped, for example, paste-like thrombi composed predominantly of a protein called fibrin or thrombi associated with the agglomeration of blood platelets may have already been formed, or new thrombi may be formed, by the action of coagulation factors in the blood. The thrombi formed in the blood vessels increase a non-perfusion area that is not perfused with a perfusate. In this case, the compatibility of the organ with the recipient and the rate of engraftment of the organ after organ transplantation may deteriorate.

Meanwhile, for example, Patent Literatures 1 to 3 disclose techniques for dissolving thrombi or preventing the formation of new thrombi by using nattokinase to suppress the activation of coagulation factors in the blood or by activating plasmin by the action of protease that belongs to factors of the fibrinolytic system.

### Citation List

### Patent Literature

Patent Literature 1: Japanese Patent Application Laid-Open No. 2016-013976
Patent Literature 2: Japanese Patent Application Laid-Open No. 2016-504287
Patent Literature 3: Japanese Patent Application Laid-Open No. 2017-119683

### Summary of Invention

### Technical Problem

However, the methods disclosed in Patent Literatures 1 to 3 are premised on oral administration to patients and are not intended to be applied directly to the method of organ preservation for use in transplantation or other purposes.

The present invention has been made in light of such circumstances, and it is an object of the present invention to provide a technique that is able to dissolve thrombi in blood vessels of an organ or prevent the formation of new thrombi even in cases such as where the organ is preserved ex vivo for a long time or where a considerable amount of time has already elapsed since the bloodstream in vivo was stopped.

### Solution to Problem

To solve the problems described above, a first aspect according to the present application is a perfusate for use in organ perfusion. The perfusate includes a biocompatible solution and a serine protease and has an osmotic pressure ratio higher than or equal to 1 and lower than or equal to 5 relative to a saline solution.

A second aspect of the present application is the perfusate according to the first aspect, in which the biocompatible solution contains at least one of a culture medium, an L-15 culture medium, an ETK solution, an HTK solution, a saline solution, an infusion solution, or a UW solution.

A third aspect of the present application is the perfusate according to the first or second aspect, in which the serine protease is predominantly composed of at least one of plasmin, thrombin, urokinase, alteplase, streptokinase, granzyme B, or nattokinase.

A fourth aspect of the present application is the perfusate according to any one of the first to third aspects, in which a concentration of the serine protease in the perfusate is higher than or equal to 0.01 mg/mL and lower than or equal to 100 mg/mL.

A fifth aspect of the present application is a perfusion method for perfusing an organ with a perfusate. The perfusion method includes a) cleaning a blood vessel of the organ by causing a first perfusate to flow into the blood vessel of the organ and letting the first perfusate out of the organ through the blood vessel, and b) after the operation a), preserving the organ while causing the second perfusate to flow into the blood vessel and letting the second perfusate out of the organ through the blood vessel. The first perfusate and the second perfusate each contain a biocompatible solution and a serine protease. The first perfusate and the second perfusate each have an osmotic pressure ratio higher than or equal to 1 and lower than or equal to 5 relative to a saline solution, and a concentration of the serine protease in the second perfusate is lower than a concentration of the serine protease in the first perfusate.

A sixth aspect of the present application is the perfusion method according to the fifth aspect, in which the biocompatible solution contains at least one of a culture medium, an L-15 culture medium, an ETK solution, an HTK solution, a saline solution, an infusion solution, or a UW solution.

A seventh aspect of the present application is the perfusion method according to the fifth or sixth aspect, in which the serine protease is predominantly composed of at least one of plasmin, thrombin, urokinase, alteplase, streptokinase, granzyme B, or nattokinase.

An eighth aspect of the present application is the perfusion method according to any one of the fifth to seventh aspects, in which a concentration of the serine protease in the second perfusate is higher than or equal to 0.01 mg/mL and lower than or equal to 100 mg/mL.

### Advantageous Effects of Invention

According to the first to eighth aspects of the present application, it is possible to dissolve thrombi in blood vessels of an organ or prevent the formation of new thrombi by the effect of the serine protease. This improves the compatibility of the organ with the recipient and the rate of engraftment of the organ after organ transplantation. It is also possible to suppress damage to cells in the organ, which may be caused by an outflow of moisture in the organ due to an excessive increase in the osmotic pressure of the perfusate or caused by an inflow of moisture into the organ from outside due to an excessive decrease in the osmotic pressure of the perfusate.

### Brief Description of Drawings

Fig. 1 is a schematic view illustrating a configuration of a perfusion device;
Fig. 2 is a cross-sectional view of a storage container;
Fig. 3 is a graph showing the result of verifying the effect of using a perfusate;
Fig. 4 is a graph showing the result of verifying the relationship between the concentration of a serine protease in the perfusate and the rate of dissolution of thrombi;
Fig. 5 is a graph showing the result of verifying the relationship between the concentration of the serine protease in the perfusate and the rate of dissolution of thrombi;
Fig. 6 is a flowchart of a procedure for a liver removal operation and perfusion treatment processes;
Fig. 7 is a schematic view illustrating a configuration of a simple perfusion device; and
Fig. 8 is a flowchart of a procedure for a liver transplant operation and perfusion treatment processes.

### Description of Embodiments

Hereinafter, one embodiment of the present invention will be described with reference to the accompany drawings, using, for example, perfusion treatment performed until an organ of a donor such as a liver is transplanted into a recipient. In the present application, "donors" and "recipients" may be humans, or may be non-human animals. That is, "organs" according to the present application may be human organs, or may be organs of non-human animals. The non-human animals may be rodents such as mice and rats, ungulates such as pigs, goats, and sheep, non-human primates such as chimpanzees, or other non-human mammals, or may also be nonmammalian animals. In the following description, perfusion treatment is performed in both cases where a target organ is in a living body and where the organ has been taken out of a living body.

### 1. One Embodiment

### 1-1. Configuration of Perfusion Device

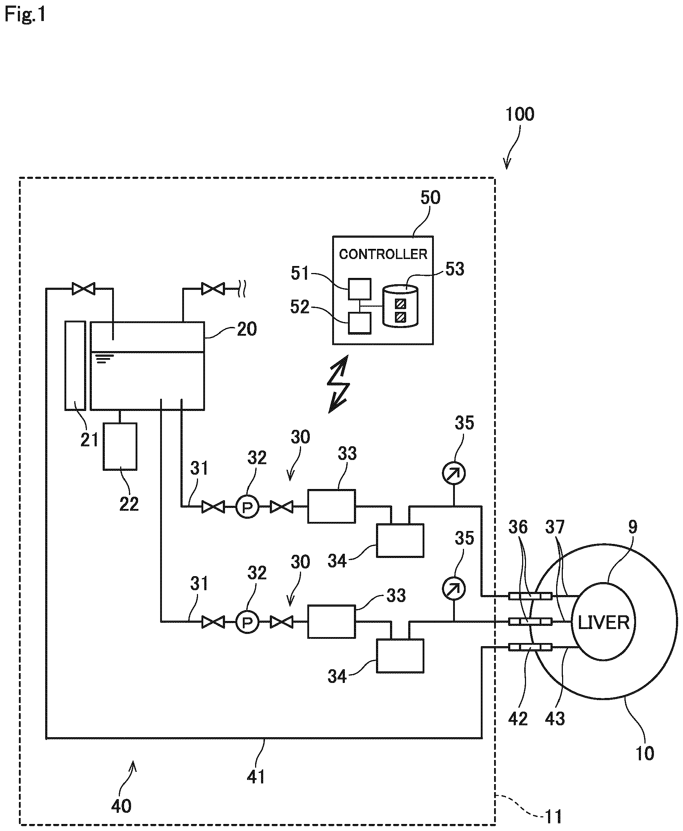

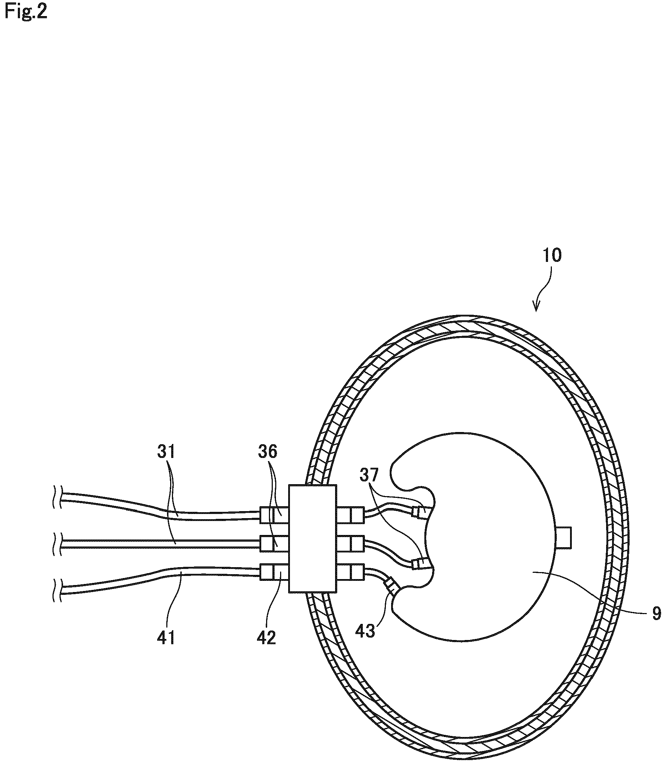

First, a perfusion device 100 for use in perfusion treatment according to one embodiment of the present invention will be described with reference to Fig. 1. Fig. 1 is a schematic view illustrating a configuration of the perfusion device 100. Fig. 2 is a sectional view of a storage container 10, which will be described later.

The perfusion device 100 is a device for providing perfusion treatment for an organ of a donor such as a liver until the organ is transplanted into a recipient. The perfusion device 100 supplies a perfusate to the organ for perfusion treatment. The following description is given of a case where an organ to be subjected to perfusion treatment is a liver 9 for transplantation.

As illustrated in Fig. 1, the perfusion device 100 includes the storage container 10 and a device body 11.

During perfusion treatment by the perfusion device 100, the liver 9 is stored in the storage container 10. The storage container 10 is an organ container capable of storing an organ such as the liver 9 and a preservation solution for immersion of an organ such as the liver 9. For example, the storage container 10 may be a cup-shaped (closed-end tubular) container.

As illustrated in Fig. 1, the perfusion device 100 includes a reservoir 20, two (two lines of) perfusate inflow parts 30, a perfusate outflow part 40, and a controller 50. The number of perfusate inflow parts 30 and the number of perfusate outflow part 40 each may be one (one line) or two (two lines) depending on perfusion conditions and the type of the organ to be subjected to perfusion.

The reservoir 20 is a container that stores a perfusate. A temperature control mechanism 21 and a gas exchange mechanism 22 are provided around the reservoir 20. The composition of the perfusate will be described later in detail.

The temperature control mechanism 21 controls the temperature of the perfusate stored in the reservoir 20. The gas exchange mechanism 22 supplies a gas such as oxygen to the perfusate stored in the reservoir 20 so as to dissolve the gas in the perfusate. Note that the gas exchange mechanism 22 may be interposed in later described inlet piping 31 of each perfusate inflow part 30.

The perfusate inflow parts 30 supply the perfusate from the reservoir 20 to the liver 9. Each of the two (two lines of) perfusate inflow parts 30 includes the inlet piping 31, a pump 32, a temperature control unit 33, a deaerating unit 34, a pressure indicator 35, an inflow connecting pipe 36, and an inflow cannula 37. The pump 32, the temperature control unit 33, the deaerating unit 34, and the pressure indicator 35 are interposed in the inlet piping 31.

One end of the inlet piping 31 is connected to the reservoir 20. The other end of the inlet piping 31 is connected to a blood vessel of the liver 9 via the inflow connecting pipe 36 and the inflow cannula 37 during perfusion treatment. This allows the perfusate to be supplied from the reservoir 20 to the blood vessel of the liver 9.

The pump 32 produces a flow of the perfusate from the reservoir 20 to the liver 9 in the inlet piping 31. For example, the pump 32 may be provided with a brushless motor. The temperature control unit 33 controls the temperature of the perfusate in the inlet piping 31. For example, the temperature control unit 33 immerses part of the inlet piping 31 in a liquid whose temperature is set to, for example, 4°C, 20°C, or 37°C to control the temperature of the perfusate in the inlet piping 31 at the set temperature. It is, however, noted that the mechanism for controlling the temperature of the perfusate may not be provided, or may be provided in only either of the reservoir 20 and the inlet piping 31. The deaerating unit 34 removes gas components of the perfusate in the inlet piping 31. The pressure indicator 35 measures the pressure of the perfusate in the inlet piping 31.

As illustrated in Fig. 2, the inflow connecting pipes 36 are cylindrical members. The inflow connecting pipes 36 are fixed to the storage container 10 while penetrating the storage container 10. One ends of the inflow connecting pipes 36 are open to the inside of the storage container 10. The other ends of the inflow connecting pipes 36 are open to the outside of the storage container 10.

In the case of the liver 9 in a living body, the blood is generally supplied from the portal vein and the hepatic artery. The portal vein is a thick vein that supplies, to the liver 9, venous blood that contains a large amount of nutrients having circulated through the bowel or the spleen. The hepatic artery supplies arterial blood that contains a large amount of oxygen from the main artery to the liver 9. These blood join in the liver 9, flow in capillaries in the liver 9, and then return to the heart through the suprahepatic inferior vena cava (SH-IVC).

During perfusion treatment, the inflow cannula 37 are connected respectively to the portal vein and hepatic artery of the liver 9 of the donor. The other ends of the inflow cannula 37 are connected to the one ends of the inflow connecting pipes 36 inside the storage container 10. Each inlet piping 31 is connected to the reservoir 20 and to the other end of the inflow connecting pipe 36 outside the storage container 10. The inlet piping 31 according to the present embodiment also be able to be directly connected to the inflow cannula 37 without the intervention of the inflow connecting pipe 36.

As described above, in the present embodiment, the perfusate is supplied from the two lines of perfusate inflow parts 30 to the liver 9, with the inlet piping 31 of one line connected to the portal vein of the liver 9, and the inlet piping 31 of the other line connected to the hepatic artery of the liver 9. The hepatic artery of the liver 9 in a living body is in the arterial system, and the pressure of the blood in the hepatic artery is high. In contrast, the portal vein is in the venous system, and the pressure of the blood in the portal vein is much lower than the pressure of the blood in the hepatic artery. The aforementioned configuration of the present embodiment enables individually setting the pressure of the perfusate supplied to the hepatic artery and the pressure of the perfusate supplied to the portal vein even during perfusion treatment.

The perfusate outflow part 40 causes the perfusate to be discharged from the liver 9. The perfusate outflow part 40 includes outlet piping 41, an outflow connecting pipe 42, and an outflow cannula 43.

One end of the outlet piping 41 is connected to the reservoir 20. The other end of the outlet piping 41 is connected to a blood vessel of the liver 9 via the outflow connecting pipe 42 and the outflow cannula 43 during perfusion treatment.

The outflow connecting pipe 42 is a cylindrical member. The outflow connecting pipe 42 is fixed to the storage container 10 while penetrating the storage container 10. One end of the outflow connecting pipe 42 is open to the inside of the storage container 10. The other end of the outflow connecting pipe 42 is open to the outside of the storage container 10.

During perfusion treatment, the cylindrical outflow cannula 43 is connected to the suprahepatic inferior vena cava (SH-IVC) or infrahepatic inferior vena cava (IH-IVC) of the liver 9 of the donor. The other end of the outflow cannula 43 is connected to the one end of the outflow connecting pipe 42 inside the storage container 10. The outlet piping 41 is connected to the reservoir 20 and to the other end of the outflow connecting pipe 42 outside the storage container 10. This allows the perfusate discharged from the suprahepatic inferior vena cava (SH-IVC) or infrahepatic inferior vena cava (IH-IVC) of the liver 9 to return back to the reservoir 20. The outlet piping 41 of the present embodiment also be able to be directly connected to the outflow cannula 43 without the intervention of the outflow connecting pipe 42.

As described above, the perfusate stored in the reservoir 20 is supplied to the portal vein and hepatic artery of the liver 9 via the perfusate inflow parts 30 by the power of the pumps 32, join in the liver 9, and flow in capillaries in the liver 9. Thereafter, the perfusate is discharged from the suprahepatic inferior vena cava (SH-IVC) or infrahepatic inferior vena cava (IH-IVC) and flows back to the reservoir 20 via the perfusate outflow part 40. Alternatively, the outlet piping 41 may be further provided with a pump that produces a flow of the perfusate from the liver 9 to the reservoir 20.

Although the perfusion device 100 according to the present embodiment is configured to return the perfusate discharged from the liver 9 back to the reservoir 20, the present invention is not limited to this configuration. The perfusate discharged from the liver 9 may be discarded without flowing back to the reservoir 20, or may be stored in a different container.

The reservoir 20, the perfusate inflow parts 30, and the perfusate outflow part 40 each may be provided with a measuring unit that detects pH or a specific component as appropriate. As another alternative, each inlet piping 31 and the outlet piping 41 may have a device interposed therein, such as a flowmeter or a solenoid valve that controls communication.

The controller 50 is configured to control operations of each constituent element of the perfusion device 100. As schematically illustrated in Fig. 1, the controller 50 consists of, for example, a computer that includes an arithmetic processing unit 51 such as a CPU, a memory 52 such as a RAM, and a storage 53 such as a hard disk drive.

In the perfusion treatment using the above-described perfusion device 100, there are cases such as where the organ of the donor such as the liver 9 is processed ex vivo for a long time or where the processing of the organ is started after a considerable amount of time has elapsed since the bloodstream in vivo was stopped. In this case, for example, paste-like thrombi composed predominantly of a protein called fibrin or thrombi associated with the agglomeration of blood platelets may have been formed already, or new thrombi may be formed, by the action of coagulation factors in the blood in the organ.

### 1-2. Composition of Perfusate

Next, the composition of the perfusate for use in perfusion treatment according to one embodiment of the present invention will be described in detail.

The perfusate according to one embodiment of the present invention is produced by primarily adding a serine protease to an aqueous or electrolytic solution having physiological activity or biocompatibility. Hereinafter, the aqueous or electrolytic solution having physiological activity or biocompatibility is referred to as a "biocompatible solution." One example of the biocompatible solution is a commercially available L-15 culture medium. Alternatively, the biocompatible solution may also be a culture medium, an ETK solution, an HTK solution, a saline solution, an infusion solution, an UW solution, or any combination of these solutions.

The serine protease according to the present embodiment is predominantly composed of nattokinase. In general, Bacillus natto, which is a kind of Bacillus subtilis in Natto, is known to produce a proteolytic enzyme called nattokinase. It has conventionally been reported that nattokinase in a living body has the effect of decomposing fibrin, which is a major component of thrombi, and improves blood circulation in a living body through ingestion. Besides, nattokinase can be introduced at low cost because it is derived from food and easy to extract.

Alternatively, the major component of the serine protease may also be plasmin, thrombin, urokinase, alteplase, streptokinase, granzyme B, or any mixture of them. That is, the serine protease may be predominantly composed of at least one of plasmin, thrombin, urokinase, alteplase, streptokinase, granzyme B, or nattokinase.

It is more preferable that an oxygen carrier is added to the perfusate. The oxygen carrier contained in the perfusate suppresses the occurrence of disorders of the liver 9 for transplantation and improves the percentage of success of liver transplantation. Examples of the oxygen carrier that can be used in the present invention include red blood cells and artificial red blood cells. The red blood cells to be added to the perfusate of the present invention are preferably red blood cells of the blood type that can be transfused to the donor or the recipient, and more preferably red blood cells originated from either the donor or the recipient. The artificial red blood cells to be added to the perfusate of the present invention may be molecules that have the function of carrying oxygen, or may for example be perfluorocarbon or hemoglobin endoplasmic reticulum. As another alternative, blood plasma or blood serum may be added to the perfusate.

The perfusate of the present embodiment has an osmotic pressure ratio adjusted to higher than or equal to 1 and lower than or equal to 5 relative to the saline solution. This reduces damage to cells in the liver 9 caused by an outflow of moisture in the liver 9 due to an excessive increase in the osmotic pressure of the perfusate, and also reduces damage to cells in the liver 9 caused by an inflow of moisture into the liver 9 from outside due to an excessive decrease in the osmotic pressure of the perfusate.

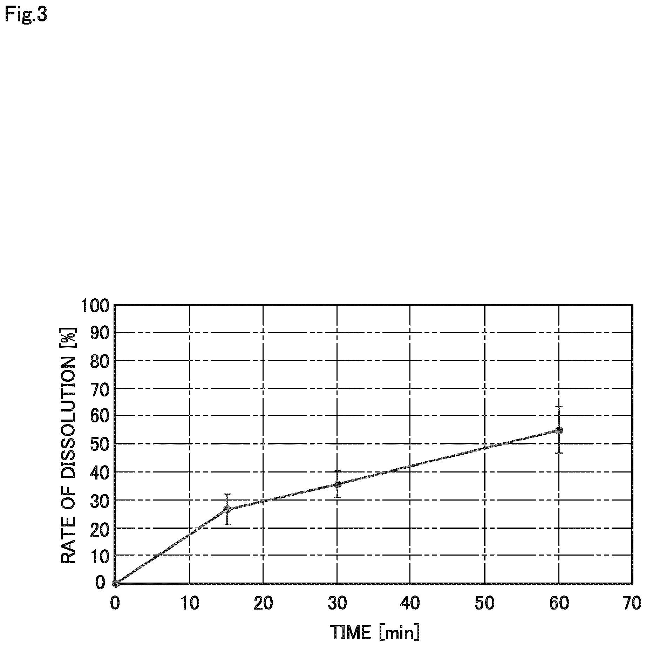

Fig. 3 is a graph showing the result of verifying the effect of using such a perfusate. Specifically, Fig. 3 shows the result of verifying the rate of dissolution of thrombi in the blood taken out of a pig's body when a perfusate made by adding nattokinase and 12.5% of human serum albumin to an L-15 culture medium, which serves as a biocompatible solution, is added to the thrombi. In the graph in Fig. 3, the horizontal axis indicates the time elapsed since the addition of the perfusate to the thrombi, and the vertical axis indicates the rate of dissolution of thrombi. Seven patterns of the concentration of nattokinase in the perfusate, ranging from 0.025 to 250 mg/mL, have been selected for verification. The perfusate has an osmotic pressure ratio of approximately 3 relative to the saline solution (with an osmotic pressure of 280 to 299 mmol/kg).

It has been confirmed as illustrated in Fig. 3 that the use of the aforementioned perfusate increases the rate of dissolution of thrombi with increasing elapsed time since the addition of the perfusate to the thrombi, and that the rate of dissolution of thrombi reaches approximately 60% after the lapse of 60 minutes. This is considered because the serine protease such as nattokinase works to selectively accelerate the hydrolytic degradation (disconnection) of peptide bonds where a large number of amino acids are condensed together, in the protein serving as the major component of the thrombi.

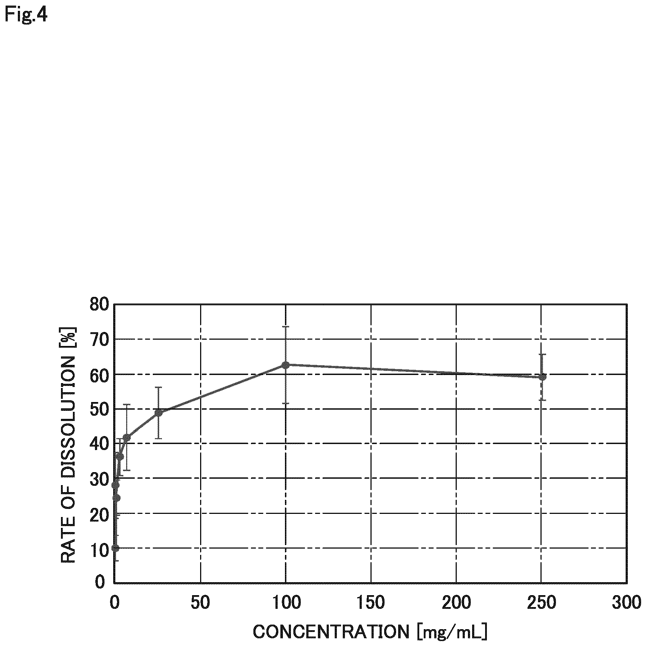

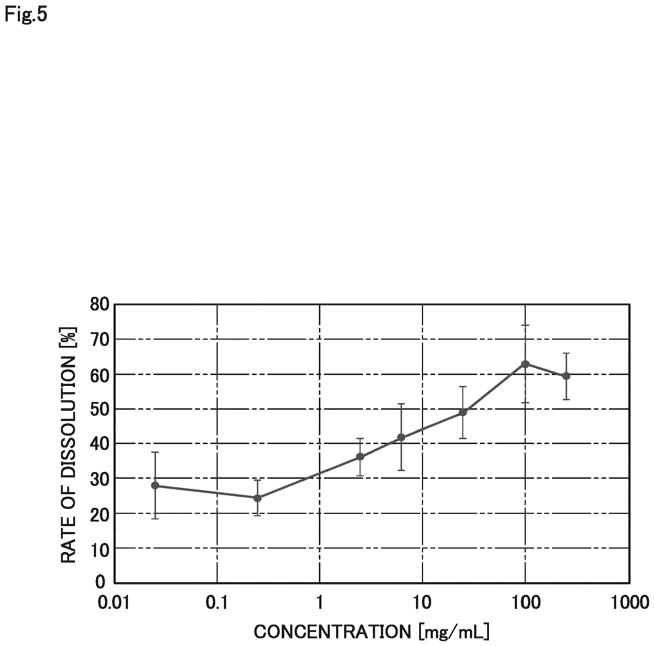

Figs. 4 and 5 are graphs each showing the result of verifying the relationship between the concentration of the serine protease in the aforementioned perfusate and the rate of dissolution of thrombi. Specifically, Figs. 4 and 5 each show the result of verifying the rate of dissolution of thrombi in the blood taken out of a pig's body when the seven types of the perfusate were respectively added to the thrombi, the seven types of the perfusate being made by adding nattokinase and 12.5% of human serum albumin to a L-15 culture medium, which serves as a biocompatible solution, while changing the amount of nattokinase. In the graphs in Figs. 4 and 5, the horizontal axis indicates the concentration of the serine protease in the perfusate, and the vertical axis indicates the rate of dissolution of thrombi. In Fig. 5, the result of the verification in Fig. 4 is plotted on an irregular scale on the horizontal axis. The perfusate has an osmotic pressure ratio of approximately 3 relative to the saline solution (with an osmotic pressure of 280 to 299 mmol/kg). Figs. 4 and 5 also show the results of verifying the rate of dissolution of thrombi after the lapse of 15 minutes since the seven types of the perfusate were respectively added to the thrombi.

It has been confirmed as shown in Figs. 4 and 5 that, when the concentration of nattokinase in the perfusate is approximately less than or equal to 100 mg/mL, the rate of dissolution of thrombi generally rises with increasing concentration of nattokinase, and when the concentration of nattokinase exceeds approximately 100 mg/mL, conversely, the rate of dissolution of thrombi drops. Meanwhile, it has also been confirmed that even when the concentration of nattokinase in the perfusate is low in the order of approximately 0.01 mg/mL to 0.1 mg/mL, the rate of dissolution of thrombi is maintained to a certain degree. That is, the concentration of the serine protease in the perfusate is desirably set to be approximately higher than or equal to 0.01 mg/mL and approximately lower than or equal to 100 mg/mL.

### 1-3. Procedure of Perfusion Treatment

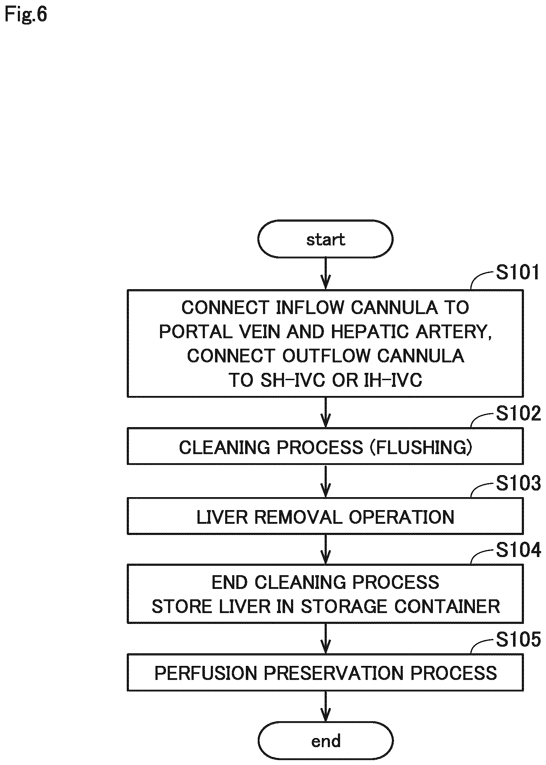

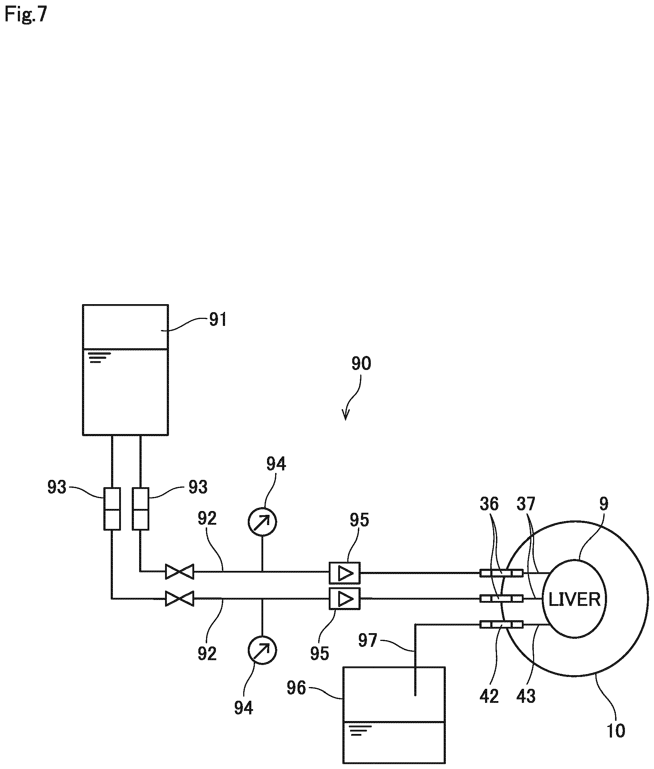

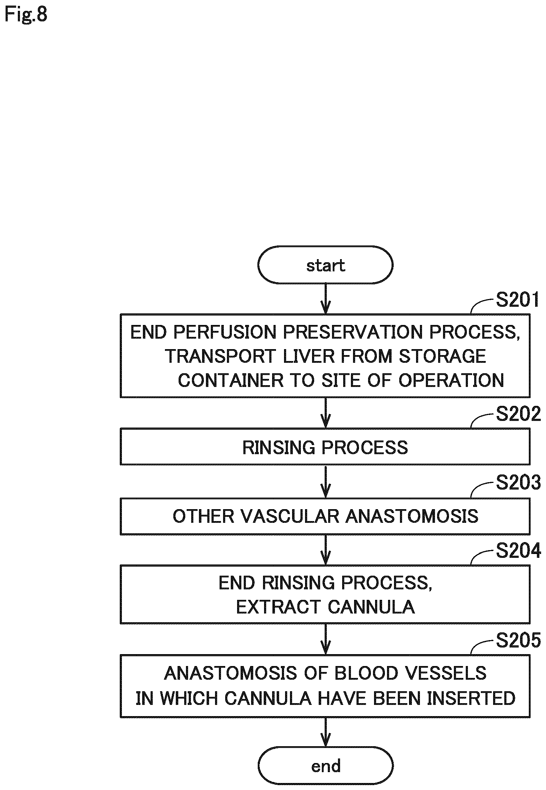

Next, procedures of the operation of removing the liver 9 and the operation of transplanting the liver 9, and perfusion treatment to be provided during the removal and transplant operations of the present embodiment will be described with reference to Figs. 6 to 8. Fig. 6 is a flowchart illustrating the procedure for the operation of removing the liver 9 and perfusion treatment processes. Fig. 7 is a schematic view illustrating a configuration of a simple perfusion device 90, which will be described later. Fig. 8 is a flowchart illustrating the procedure for the operation of transplanting the liver 9 and perfusion treatment processes. As will be described below, the perfusion treatment is roughly divided into a "cleaning process (flushing)," a "perfusion preservation process," and a "rinsing process."

Two types of perfusate, first perfusate and second perfusate, are each used as the perfusate. The first perfusate and the second perfusate each contain a biocompatible solution and a serine protease and are adjusted to have an osmotic pressure ratio higher than or equal to 1 and lower than or equal 5 relative to the saline solution. The concentration of the serine protease in the second perfusate is higher than or equal to 0.01 mg/mL and lower than or equal to 100 mg/mL and adjusted to be lower than the concentration of the serine protease in the first perfusate. It is, however, noted that the concentration of the serine protease in the first perfusate may be slightly higher than 100 mg/mL.

In the removal operation, as illustrated in Fig. 6, the portal vein and hepatic artery of the liver 9 are severed in the abdominal cavity of the donor and connected respectively to the one ends of the inflow cannula 37. The suprahepatic inferior vena cava (SH-IVC) or infrahepatic inferior vena cava (IH-IVC) of the liver 9 is also severed in the abdominal cavity of the donor and connected to the one end of the outflow cannula 43 (step S101).

Then, the aforementioned perfusion device 100 is used to directly connect the other ends of the inflow cannula 37 to the two lines of inlet piping 31. The other end of the outflow cannula 43 is also directly connected to the outlet piping 41. Then, the perfusion device 100 is driven while storing the first perfusate in the reservoir 20 to allow the first perfusate to flow into the portal vein and hepatic artery of the liver 9. Accordingly, the first perfusate supplied to the portal vein and the first perfusate supplied to the hepatic artery join in the liver 9 and flow in capillaries in the liver 9. Thereafter, the first perfusate is discharged from the suprahepatic inferior vena cava (SH-IVC) or the infrahepatic inferior vena cava (IH-IVC) and flows back to the reservoir 20 via the perfusate outflow part 40 (step S102). This process is in general called the "cleaning process (flushing)." At this time, the flow of the first perfusate containing a high concentration of the serine protease through the blood vessels enables dissolving thrombi already formed in the blood vessels and preventing the formation of new thrombi as well as sufficiently removing waste products or the like in the blood vessels and cleaning the blood vessels.

However, the "cleaning process (flushing)" does not necessarily have to include connecting the outlet piping 41 and the reservoir 20. That is, the first perfusate discharged from the suprahepatic inferior vena cava (SH-IVC) or the infrahepatic inferior vena cava (IH-IVC) may be discarded in the downstream of the outflow cannula 43 and the outlet piping 41, or may be stored in a different container. In particular, it is desirable that the first perfusate immediately after starting to be discharged from the suprahepatic inferior vena cava (SH-IVC) or the infrahepatic inferior vena cava (IH-IVC) should be discarded.

Next, the other blood vessels extending from the liver 9 are severed to remove the liver 9 from the donor (step S103).

Thereafter, the driving of the perfusion device 100 and the supply of the first perfusate are stopped to end the "cleaning process (flushing)." Then, the connection between the inflow cannula 37 and the two lines of inlet piping 31 and the connection between the outflow cannula 43 and the outlet piping 41 are disconnected respectively, and the liver 9 is stored in the storage container 10 (step S104).

Then, the inflow cannula 37 are connected to the one ends of the inflow connecting pipes 36 fixed to the storage container 10, and the other ends of the inflow connecting pipes 36 are connected to the two lines of inlet piping 31. The outflow cannula 43 is connected to the one end of the outflow connecting pipe 42 fixed to the storage container 10, and the other end of the outflow connecting pipe 42 is connected to the outlet piping 41. Then, the perfusion device 100 is driven while storing the second perfusate in the reservoir 20, so as to cause the second perfusate to flow into the portal vein and hepatic artery of the liver 9. Accordingly, the second perfusate supplied to the portal vein and the second perfusate supplied to the hepatic artery join in the liver 9 and flow in capillaries in the liver 9. Thereafter, the second perfusate is discharged from the suprahepatic inferior vena cava (SH-IVC) or the infrahepatic inferior vena cava (IH-IVC) and flows back to the reservoir 20 via the perfusate outflow part 40 (step S105). This process is in general called the "perfusion preservation process." At this time, the flow of the second perfusate through the blood vessels enables dissolving thrombi already formed in the blood vessel and preventing the formation of new thrombi. As a result, the compatibility of the transplanted liver 9 with the recipient and the rate of engraftment of the liver 9 are improved. Through these processes, the operation of removing the liver 9 from the donor is completed.

In steps S101 to S105 described above, treatments that are usually conducted in the procedure for removing the liver 9 for transplantation from the donor are provided appropriately as necessary by a surgeon or other specialists. For example, treatments such as removal of connective tissues, separation of blood vessels, temporary ligation or clamping of blood vessels for severing of blood vessels, blockage and severing of the bile duct, the application of a blood anticoagulating agent to the liver 9 for transplantation, and hemostatic treatment of surgical sites may be provided appropriately as necessary. Moreover, components other than those described above may be added to each of the first perfusate and the second perfusate. For example, a nutrient for activating cells in the liver 9 may be further added to the second perfusate.

After the removal of the liver 9 from the donor is completed, the perfusion device 100 performs perfusion until the organ is started to be transported to the recipient. Thereafter, during the transport of the organ to the recipient, simple perfusion is performed by the simple perfusion device 90 as illustrated in Fig. 7 while transporting the liver 9. Accordingly, it is possible even during the transport to dissolve thrombi in the blood vessels of the liver 9 and to prevent the formation of new thrombi. In the case where there is some time before the transplant operation is conducted for the recipient after the arrival of the liver 9 at the hospital or in an operating room on the recipient side or where there is a need to recover the function of the liver 9 by perfusion, the perfusion device 100 performs perfusion again.

As illustrated in Fig. 7, the simple perfusion device 90 includes a bag reservoir 91, lines of inlet piping 92 that provide connection between the bag reservoir 91 and the inflow connecting pipes 36, air traps 93, pressure indicators 94, flowmeters 95, a waste water disposal tank 96, and drainage piping 97 that provides connection between the outflow connecting pipe 42 and the waste water disposal tank 96. The air traps 93, the pressure indicators 94, and the flowmeters 95 are interposed in the lines of inlet piping 92.

The bag reservoir 91 is a an intravenous drip bag shaped container filled with the perfusate (second perfusate). The bag reservoir 91 is arranged above the storage container 10, so that the second perfusate flows from the bag reservoir 91 into the liver 9 through the inlet piping 92, the inflow connecting pipes 36, and the inflow cannula 37 as in the case of intravenous drip injection.

The second perfusate flowing from the inflow cannula 37 into the liver 9 flows through the liver 9 in the same manner and is discharged from the outflow cannula 43 to the waste water disposal tank 96 through the outflow connecting pipe 42 and the drainage piping 97. The waste water disposal tank 96 is preferably arranged below the storage container 10.

The use of the simple perfusion device 90 allows the perfusion treatment of the liver 9 to be conducted in an abbreviated manner when the liver 9 is transported from the place where the removal operation from the donor has been conducted to the place where the transplant operation for the recipient is to be conducted. Accordingly, it is possible even during transport to dissolve thrombi formed in the blood vessels of the liver 9 and to prevent the formation of new thrombi.

Next, the procedure for the operation of transplanting the liver 9 and perfusion treatment processes will be described with reference to Fig. 8. As illustrated in Fig. 8, the supply of the second perfusate is first stopped to end the "perfusion preservation process." Then, the connection between the inflow cannula 37 and the inflow connecting pipes 36 and the connection between the outflow cannula 43 and the outflow connecting pipe 42 are disconnected respectively, and the liver 9 is taken out of the storage container 10 and placed in a transplantation position of the recipient (step S201).

Then, the inflow cannula 37 and the inlet piping 31 are directly connected to each other, using the aforementioned perfusion device 100. The outflow cannula 43 and the outlet piping 41 are also directly connected to each other. Then, the perfusion device 100 is driven while storing a rinsing liquid in the reservoir 20, so as to cause the rinsing liquid to flow into the portal vein and hepatic artery of the liver 9 (step S202). This process is in general referred to as the "rinsing process." The rinsing liquid as used herein differs from the first perfusate and the second perfusate described above. The rinsing liquid may, for example, be a liquid with no proteolytic enzyme added thereto or with an enzyme deactivator added thereto. For example, the rinsing liquid may be a perfusate that contains no serine protease. As another alternative, the rinsing liquid may have a reducing agent added thereto in order to reduce damage to vascular endothelial cells. The rinsing liquid supplied to the portal vein and the rinsing liquid supplied to the hepatic artery join in the liver 9 and flow in capillaries in the liver 9. Thereafter, the rinsing liquid is discharged from the suprahepatic inferior vena cava (SH-IVC) or the infrahepatic inferior vena cava (IH-IVC) and flows back to the reservoir 20 via the perfusate outflow part 40. Accordingly, the first perfusate and the second perfusate that remain in the blood vessels are removed. As a result, the compatibility of the liver 9 with the recipient and the rate of engraftment of the liver 9 are further improved. Note that the "rinsing process" may be omitted.

Then, blood vessels of the liver 9 into which neither the inflow cannula 37 nor the outflow cannula 43 are inserted are anastomosed to the corresponding blood vessels of the recipient (step S203).

Thereafter, the driving of the perfusion device 100 and the supply of the rinsing liquid are stopped to end the "rinsing process." Next, the inflow cannula 37 and the outflow cannula 43 are removed from the liver 9 (step S204). Then, the blood vessels connected to the inflow cannula 37 and the outflow cannula 43 are anastomosed to the corresponding blood vessels of the recipient respectively (S205). Through the processes described above, the operation of transplanting the liver 9 into the recipient is completed.

In steps S201 to S205 described above, treatments that are usually conducted in the procedure for transplanting the liver 9 for transplantation into the recipient are provided appropriately as necessary by a surgeon or other specialists. For example, treatments such as removal of connective tissues, separation of blood vessels, temporary ligation or clamping of blood vessels for severing of blood vessels, blockage and severing of the bile duct, the application of a blood anticoagulating agent to the liver 9 for transplantation, and hemostatic treatment of surgical sites may be provided appropriately as necessary.

### 2. Variations

Although one embodiment of the present invention has been described thus far, the present invention is not intended to be limited to the above-described embodiment.

In the above-described embodiment, the perfusate is supplied from both of the portal vein and the hepatic artery. Ordinarily, about 30% of the blood flow to the liver 9 comes from the hepatic artery acting as the vasa vasorum of the liver 9, and about 70% comes from the portal vein acting as a functional blood vessel of the liver 9. In order to preserve the liver 9 in excellent conditions during perfusion treatment, it is preferable that the perfusate with oxygen added thereto is supplied to the hepatic artery acting as the vasa vasorum. However, supplying the perfusate only to the hepatic artery reduces the flow rate of the perfusate that can be supplied to the liver 9 and unfavorably allows easy formation of thrombi in the portal vein. In view of this, it is preferable that the perfusate is supplied to both of the hepatic artery and the portal vein during perfusion treatment. Accordingly, it is possible as in the above-described embodiment to put the liver 9 in a more favorable condition by supplying the perfusate to the liver 9 through the two perfusate inflow paths.

As described above, it is preferable that oxygen is added to at least the perfusate supplied to the hepatic artery. In the case where the reservoir 20 includes the gas exchange mechanism 22 as in the above-described embodiment (see Fig. 1), oxygen can be added to the perfusate stored in the reservoir 20. Accordingly, if the two perfusate inflow paths are prepared as in the example in Fig. 1, the perfusate with oxygen added thereto is supplied to both of the hepatic artery and the portal vein. The present invention is, however, not limited to this example. For example, instead of including the gas exchange mechanism in the reservoir 20, an oxygenation mechanism may be interposed in the perfusate inflow paths connected to each of the hepatic artery and the portal vein. Such a mechanism for adding oxygen to the perfusate may be provided in only one of the perfusate inflow paths connected to the hepatic artery and the portal vein, or may be provided in both of the perfusate inflow paths.

In the case where the reservoir 20 includes the gas exchange mechanism 22 as in the above-described embodiment, the perfusate to be supplied to the hepatic artery and the perfusate to be supplied to the portal vein both have the same amount of oxygen added. In contrast, if an oxygenation mechanism is interposed in each perfusate inflow path, it is possible to individually set the amount of oxygen added to the perfusate supplied to the hepatic artery and the amount of oxygen added to the perfusate supplied to the portal vein.

As the number of perfusate inflow paths or perfusate outflow paths increases, the size of the perfusion device as a whole increases as in the above-described embodiment (see Fig. 1). In view of this, only one perfusate outflow path is provided in the example in Fig. 1. The blood vessel connected to the perfusate outflow path in the liver 9 is either the suprahepatic inferior vena cava (SH-IVC) or the infrahepatic inferior vena cava (IH-IVC). Since these veins form parts of the same vein, even if only one of the veins is connected to the perfusate outflow path, a reduction in the efficiency of discharging the perfusate is slight as compared with the case where both of the veins are connected to perfusate outflow paths. Accordingly, if the perfusion device 100 includes two perfusate inflow paths connected respectively to the hepatic artery and the portal vein and one perfusate outflow path as in the example in Fig. 1, it is possible to suppress upsizing of the perfusion device 100 as much as possible while putting the liver 9 in a more favorable condition. However, the number of perfusate inflow paths and the number of perfusate outflow paths each may be one or two. As another alternative, the number of perfusate inflow paths and the number of and perfusate outflow paths each may be three or more.

In the embodiment and variations described above, irrespective of the presence or absence of thrombi, the perfusate that contains a serine protease acting as a proteolytic enzyme is used for perfusion in the "cleaning process (flushing)" and the "perfusion preservation process." However, in the case where there are no thrombi in the blood vessels or where there are no non-perfusion areas where the perfusate does not reach, a perfusate that does not contain a serine protease or that contains an extremely low concentration of serine protease may be used for perfusion in order to maximize the flow rate of the perfusate while reducing the pressure in the portal vein and the pressure in the hepatic artery. For example, a perfusate that does not contain a serine protease or that contains an extremely low concentration of serine protease may be used for perfusion in order to maximize the flow rate of the perfusate while reducing the pressure in the portal vein to 10 mmHg or less and the pressure in the hepatic artery to 100 mmHg or less.

In the embodiment and variations described above, the organ to be preserved is a liver, but the present invention is not limited thereto. The organ to be preserved may be any other organ such as a kidney.

That is, the present invention relates to a perfusate for use in organ perfusion, and the perfusate may contain a biocompatible solution and a serine protease and has an osmotic pressure ratio higher than or equal to 1 and lower than or equal to 5 relative to the saline solution.

The present invention also relates to a perfusion method for perfusing an organ with a perfusate. This perfusion method includes a) a cleaning process of cleaning a blood vessel of the organ by causing a first perfusate to flow into the blood vessel and letting the first perfusate out of the organ through the blood vessel, and b) a preservation process of preserving the organ after the process a) by causing a second perfusate to flow into the blood vessel and letting the second perfusate out of the organ through the blood vessel. The first perfusate and the second perfusate each contain a biocompatible solution and a serine protease and have an osmotic pressure ratio higher than or equal to 1 and lower than or equal to 5 relative to the saline solution. The concentration of the serine protease in the second perfusate may be set to be lower than the concentration of the serine protease in the first perfusate.

The configurations of the embodiment and variations described above may be appropriately combined as long as there are no mutual inconsistencies.

### Reference Signs List

- 9: Liver
- 10: Storage container
- 11: Device body
- 20: Reservoir
- 21: Temperature control mechanism
- 22: Gas exchange mechanism
- 30: Perfusate inflow part
- 31: Inlet piping
- 32: Pump
- 36: Inflow connecting pipe
- 37: Inflow cannula
- 40: Perfusate outflow part
- 41: Outlet piping
- 42: Outflow connecting pipe
- 43: Outflow cannula
- 50: Controller
- 90: Simple perfusion device
- 91: Bag reservoir
- 92: Inlet piping
- 96: Waste water disposal tank
- 97: Drainage piping
- 100: Perfusion device

## Claims

1. A perfusate for use in perfusion of an organ, the perfusate comprising:
a biocompatible solution; and
a serine protease,
wherein said perfusate has an osmotic pressure ratio higher than or equal to 1 and lower than or equal to 5 relative to a saline solution.

2. The perfusate according to claim 1, wherein
said biocompatible solution contains at least one of a culture medium, an L-15 culture medium, an ETK solution, an HTK solution, a saline solution, an infusion solution, or a UW solution.

3. The perfusate according to claim 1 or 2, wherein
said serine protease is predominantly composed of at least one of plasmin, thrombin, urokinase, alteplase, streptokinase, granzyme B, or nattokinase.

4. The perfusate according to any one of claims 1 to 3, wherein
a concentration of said serine protease in said perfusate is higher than or equal to 0.01 mg/mL and lower than or equal to 100 mg/mL.

5. A perfusion method for perfusing an organ with a perfusate, the perfusion method comprising:
a) cleaning a blood vessel of said organ by causing a first perfusate to flow into said blood vessel and letting said first perfusate out of said organ through said blood vessel; and
b) after said operation a), preserving said organ while causing a second perfusate to flow into said blood vessel and letting said second perfusate out of said organ through said blood vessel,
wherein said first perfusate and said second perfusate each contain:
a biocompatible solution; and
a serine protease,
said first perfusate and said second perfusate each have an osmotic pressure ratio higher than or equal to 1 and lower than or equal to 5 relative to a saline solution, and
a concentration of said serine protease in said second perfusate is lower than a concentration of said serine protease in said first perfusate.

6. The perfusion method according to claim 5, wherein
said biocompatible solution contains at least one of a culture medium, an L-15 culture medium, an ETK solution, an HTK solution, a saline solution, an infusion solution, or a UW solution.

7. The perfusion method according to claim 5 or 6, wherein
said serine protease is predominantly composed of at least one of plasmin, thrombin, urokinase, alteplase, streptokinase, granzyme B, or nattokinase.

8. The perfusion method according to any one of claims 5 to 7, wherein
a concentration of said serine protease in said second perfusate is higher than or equal to 0.01 mg/mL and lower than or equal to 100 mg/mL.
